# EUROPÄISCHE PATENTSCHRIFT

(11) **EP 1 423 407 B1**
(45) Veröffentlichungstag und Bekanntmachung des Hinweises auf die Patenterteilung: **08.03.2006**
(21) Anmeldenummer: 01978315.8
(22) Anmeldetag: 29.08.2001
(51) Int. Cl.: C07J 13/00, A61K 31/56, A61K 31/57, A61P 5/28, A61P 5/34

(54) **4-HALOGENIERTE 17-METHYLENSTEROIDE, VERFAHREN ZU DEREN HERSTELLUNG UND DIESE VERBINDUNGEN ENTHALTENDE PHARMAZEUTISCHE ZUSAMMENSETZUNGEN**
4-HALOGENATED 17-METHYLENE STEROIDS, METHOD FOR THE PRODUCTION THEREOF AND PHARMACEUTICAL COMPOSITIONS CONTAINING THESE COMPOUNDS
4-HALOGENES STEROIDES DE 17-METHYLENE, PROCEDE PERMETTANT DE LES PRODUIRE ET COMPOSITIONS PHARMACEUTIQUES CONTENANT CES COMPOSES

(30) Priorität: 04.09.2000 DE 10043846
(43) Veröffentlichungstag der Anmeldung: 02.06.2004
(73) Patentinhaber: Schering AG, 13353 Berlin (DE)
(72) Erfinder: MENZENBACH, Bernd, 07743 Jena (DE); ELGER, Walter, 14195 Berlin (DE); DROESCHER, Peter, 99425 Weimar (DE); HILLISCH, Alexander, 07743 Jena (DE); KAUFMANN, Günter, 07743 Jena (DE); SCHWEIKERT, Hans-Udo, 53125 Bonn (DE); MÜLLER, Gerd, 07745 Jena (DE)
(74) Vertreter: Cramer, Eva-Maria
(86) Internationale Anmeldenummer: PCT/EP2001/009943
(87) Internationale Veröffentlichungsnummer: WO 2002/019971

(56) Entgegenhaltungen:
- EP-A- 0 077 040
- WO-A-02/00681
- WO-A-98/33506
- US-A- 3 146 239
- US-A- 3 232 960
- US-A- 3 661 940
- CHEMICAL ABSTRACTS, vol. 62, no. 10, 10. Mai 1965 (1965-05-10) Columbus, Ohio, US; abstract no. 11871e, K. BARNIKOL-OETTLER ET AL: "Wittig reaction of steroid ketones" XP002187567 & J. PRAKT CHEM., Bd. 27, Nr. 1-2, 1965, Seiten 18-33,
- DATABASE CA [Online] CHEMICAL ABSTRACTS SERVICE, COLUMBUS, OHIO, US; JARMAN, MICHAEL ET AL: "Evaluation of some 4-fluoro- and 4-cyano derivatives of.DELTA.4, 3-ketosteroids as inhibitors of testosterone 5.alpha.- reductase" retrieved from STN Database accession no. 122:1349 XP002187568 & J. ENZYME INHIB. (1994), 8(1), 17-23 ,
- M. CABEZA ET AL: "Synthesis and Pharmacological Evaluation of 4-Halo Progesterone Derivatives as Antiandrogen" CHEMICAL AND PHARMACEUTICAL BULLETIN, Bd. 47, Nr. 9, September 1999 (1999-09), Seiten 1232-1236, XP002187564 TOKYO JP
- LI, XUN ET AL: "Synthesis and in Vitro Evaluation of 4-Substituted N-(1,1-Dimethylethyl)-3-oxo-4-androstene-1 7.beta.-carboxamides as 5.alpha.- Reductase Inhibitors and Antiandrogens" J. MED. CHEM. (1995), 38(9), 1456-61 , XP002187565
- DATABASE CA [Online] CHEMICAL ABSTRACTS SERVICE, COLUMBUS, OHIO, US; BRUCHOVSKY, NICHOLAS ET AL: "Kinetic parameters of 5.alpha.- reductase activity in stroma and epithelium of normal, hyperplastic, and carcinomatous human prostates" retrieved from STN Database accession no. 110:5461 XP002187569 & J. CLIN. ENDOCRINOL. METAB. (1988), 67(4), 806-16 ,
- DATABASE CA [Online] CHEMICAL ABSTRACTS SERVICE, COLUMBUS, OHIO, US; GUARNA, A. ET AL: "Relationship between structure and activity of 5.alpha.- reductase inhibitors" retrieved from STN Database accession no. 123:102007 XP002187570 & INT. CONGR. SER. (1994), 1064(SEX HORMONES AND ANTIHORMONES IN ENDOCRINE DEPENDENT PATHOLOGY), 93-108 ,
- F. A. KINCL ET AL: "Progestational activity of the derivatives of pregnane" CIENCIA (MEX.), Bd. 22, Nr. 1-2, 1962, Seiten 35-37, XP002187566

## Beschreibung

Die Erfindung betrifft 17-Methylensteroide, Verfahren zu deren Herstellung und diese Verbindungen enthaltende pharmazeutische Zusammensetzungen.

Die erfindungsgemäßen Verbindungen besitzen ein Wirkprofil mit Hybridcharakter in der Weise, daß sie als Inhibitoren der 5α-Reduktase und gleichzeitig als Gestagene wirken. Sie eignen sich daher zur Behandlung von Erkrankungen, die bei Mann und Frau in bestimmten Organen und Geweben Folge erhöhter Androgenspiegel sind. In Verbindung mit anderen hormonalen Substanzen, wie einem Östrogen, Testosteron bzw. einem starken Androgen, sind die erfindungsgemäßen Verbindungen geeignet als Kontrazeptiva für die Frau und für den Mann.

Bei der Frau können erhöhte Spiegel an 5α-Dihydroprogesteron zu schweren Störungen der Befindlichkeit beim prämenstruellen Syndrom beitragen. Diese Störungen können durch Hemmung der 5α-Reduktase ebenfalls günstig beeinflußt werden. Das gleichzeitige Vorliegen einer gestagenen Wirkung führt beim männlichen und weiblichen Geschlecht zu einer Hemmung der Gonadenfunktion. Dieser Effekt ist dann erwünscht, wenn mit der Behandlung eine antifertile Wirkung oder auch eine Hemmung der Hormonsekretion der Gonade erreicht werden soll. Da bei mit einem männlichen Foetus schwangeren Frauen die Hemmung der 5α-Reduktase die Sexualentwicklung des Foeten irreversibel stören kann, ist eine mit einer antiandrogenen Therapie einhergehende Aufhebung der Fertilität sehr erwünscht. Mögliche Indikationen sind Prostataerkrankungen, Alopecie vom männlichen Typ, Akne und Hirsutismus. Bei entsprechend disponierten Frauen können die Beschwerden beim prämenstruellen Syndrom gelindert werden.

Chemical Abstracts 62:11871e beschreibt die Verbindung 4-Chloro-17-methylen-4-androsten-3-on aber beschreibt kein biologische Aktivität dieser Verbindung. WO-A-02/00681 beschreibt die Verbindung 4-Chloro-20ξ-fluoro-21-hydroxy-4,17(20)-dien-3-on, die auch im Prioritätsdokument vom 27 Juni 2000 zu finden ist. Dieses Dokument ist also Stand der Technik gemäss Artikel 54(3)(4) EPÜ. Chemical Abstracts 122:001,349 beschreibt die Verbindung 4-Fluoroprogesteron, die 5-alpha-Reduktase hemmt. Chem. Pharm. Bull. Band 47(9) S. 1232-1236 (1999) beschreibt verschiedene 4-Halo-17α-hydroxy/acyloxy-progesteron Verbindungen, die 5-alpha-Reduktase hemmen. J. Med. Chem. Band 38(9) S. 1456-1461 (1995) beschreibt die Verbindungen 4-Chloro-17β-[N-t-butyl-carbamoyl]-androst-4-en-3-on und 4-Bromo-17β-[N-t-butyl-carbamoyl]-androst-4-en-3-on, die 5-alpha-Reduktase hemmen. Chemical Abstracts 110:005,461 beschreibt die Verbindung 4-Chloro-20-hydroxymethyl-pregn-4-en-3-on, die 5-alpha-Reduktase hemmt. US-A-3,146,239 beschreibt die Verbindungen 4-Chloro-19-norprogesteron und 4-Bromo-19-norprogesteron, die Gestagenaktivität aufweisen. US-A-3,232,960 beschreibt 4-Fluoro-progesteron Verbindungen, die Gestagenaktivität aufweisen. WO-A-98/33506 beschreibt die Verbindung 20-Cyano-pregn-4,17(20)-dien-3-on, die 5-alpha-Reduktase hemmt. Ciencia (Mex) Band 22(1-2) S. 35-37 beschreibt die Verbindung Pregn-4,17(20)-dien-3-on, die geringe Gestagenaktivität aufweist. EP-A-077,040 beschreibt die Verbindung 20-Cyano-19-norpregn-4,17(20)-dien-3-on, die Gestagenaktivität aufweist.

Aufgabe der vorliegenden Erfindung ist es daher, Verbindungen bereitzustellen, die als Inhibitoren der 5α-Reduktase und gleichzeitig als Gestagene wirken.

Diese Aufgabe wird gelöst durch 17-Methylensteroide der allgemeinen Formel I 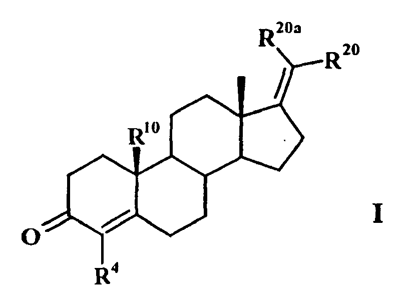
worin
R⁴ für ein Wasserstoffatom, ein Halogenatom oder ein Pseudohalogen steht,
R¹⁰ für ein Wasserstoffatom oder eine eine geradkettige oder verzweigte C₁₋₄-Alkylgruppe steht,
R²⁰ und R^{20a} unabhängig voneinander ein Wasserstoffatom, eine eine geradkettige oder verzweigte C₁₋₄-Alkyl- oder Hydroxy-C₁₋₄-alkylgruppe darstellen oder einer der Reste R²⁰ und R^{20a} ein Wasserstoffatom, eine eine geradkettige oder verzweigte C₁₋₄-Alkyl- oder Hydroxy-C₁₋₄-alkylgruppe bedeutet und der andere Rest ein Halogenatom oder ein Pseudohalogen bedeutet mit Ausnahme der Verbindungen: 4-Chloro-17-methylen-4-anchrosten-3-on und 4-choloro-20 ξ-fluoro 21-hydroxy pregna-4,17(20)-dien-3-on

R⁴ kann neben einem Wasserstoffatom ein Halogenatom, wie ein Fluor-, Chlor-, Brom-oder lodatom, oder ein Pseudohalogen, wie eine Cyanat-, Rhodanid-, Cyan- oder Azidgruppe, bedeuten, wobei ein Bromatom oder eine Cyanogruppe bevorzugt sind und ein Chloratom besonders bevorzugt ist.

R¹⁰ kann für ein Wasserstoffatom oder eine eine geradkettige oder verzweigte C₁₋₄₋Alkylgruppe stehen, wobei Beispiele für geradkettige bzw. verzweigte C₁₋₄-Alkylgruppen sind Methyl, Ethyl, Propyl, Isopropyl, Butyl, Isobutyl und tert.-Butyl sind. Bevorzugt bedeutet R¹⁰ ein Wasserstoffatom oder eine Methylgruppe.

R²⁰ und R^{20a} können zum einen unabhängig voneinander ein Wasserstoffatom, eine geradkettige oder verzweigte C₁₋₄-Alkyl- oder Hydroxy-C₁₋₄-alkylgruppe darstellen. Bevorzugt bedeuten R²⁰ und R^{20a} in diesem Fall unabhängig voneinander ein Wasserstoffatom, eine Methylgruppe oder eine Gruppe -CH₂OH.

Des weiteren kann einer der Reste R²⁰ und R^{20a} ein Wasserstoffatom, eine geradkettige oder verzweigte C₁₋₄-Alkyl- oder Hydroxy-C₁₋₄-alkylgruppe bedeuten und der andere Rest ein Halogenatom, wie ein Fluor-, Chlor-, Brom- oder lodatom, oder ein Pseudohalogen, wie eine Cyanat-, Rhodanid-, Cyan- oder Azidgruppe, bedeuten. Beispiele für geradkettige bzw. verzweigte C₁₋₄-Alkylgruppen sind die für R¹⁰ aufgeführten Gruppen. Geradkettige bzw. verzweigte Hydroxy-C₁₋₄-Alkylgruppen leiten sich von den vorstehend genannten, geradkettigen bzw. verzweigte C₁₋₄-AlkyJgruppen ab, wobei ein oder mehrere Wasserstoffatome durch Hydroxygruppen ersetzt sind.

Bevorzugt bedeutet in diesem Fall einer der Reste R²⁰ und R^{20a} ein Wasserstoffatom oder eine Methylgruppe und der andere Rest ein Halogenatom, bevorzugt ein Fluoratom, besonders bevorzugt ein Chlor- oder Bromatom, oder ein Pseudohalogen, bevorzugt eine Azido- oder Rhodanogruppe, besonders bevorzugt eine Cyanogruppe.

Im Fall von R²⁰ und R^{20a} sind Beispiele für geradkettige bzw. verzweigte C₁₋₄₋Alkylgruppen die für R¹⁰ aufgeführten Gruppen. Geradkettige bzw. verzweigte Hydroxy-C₁₋₄-Alkylgruppen leiten sich von diesen C₁₋₄-Alkylgruppen ab, wobei ein oder mehrere Wasserstoffatome durch Hydroxygruppen ersetzt sind, wie insbesondere einer Gruppe -(CH₂)n-OH mit n = 1 bis 4.

Am stärksten bevorzugt sind die folgenden Verbindungen:
1) E-17-Chlormethylen-4-chlor-estr-4-en-3-on,
2) E-17-Cyanomethylen-4-chlor-estr-4-en-3-on,
3) Z-17-Cyanomethylen-4-chlor-estr-4-en-3-on,
4) Z-20-Cyano-4-chlor-19-norpregna-4,17(20)-dien-3-on,
5) Z-4-Chlor-19-norpregna-4,17(20)-dien-3-on,
6) E-4-Chlor-19-norpregna-4,17(20)-dien-3-on,
7) E-17-Brommethylen-4-chlor-estr-4-en-3-on,
9) Z-4,20-Dichlor-19-norpregna-4,17(20)-dien-3-on,
10) Z-20-Brom-4-chlor-19-norpregna-4,17(20)-dien-3-on,
11) E-17-Chlormethylen-4-cyano-androst-4-en-3-on,
12) E-17-Chlormethylen-4-chlor-androst-4-en-3-on,
13) E-21-Hydroxy-4-chlor-19-norpregna-4,17(20)-dien-3-on und
14) Z-21-Hydroxy-4-chlor-19-norpregna-4,17(20)-dien-3-on.

Gegenstand der vorliegenden Erfindung sind ferner pharmazeutische Zusammensetzungen, die als Wirkstoffe mindestens ein 17-Methylensteroid der allgemeinen Formel I enthalten, wobei diese Zusammensetzungen gegebenenfalls geeignete Hilfs- und Trägerstoffe beinhalten, und die Verwendung dieser Verbindungen zur Herstellung von Arzneimitteln, insbesondere für die nachstehenden Indikationen.

Die zu verabreichende Menge einer Verbindung der allgemeinen Formel 1 schwankt innerhalb eines weiten Bereichs und kann jede wirksame Menge abdecken. In Abhängigkeit des zu behandelnden Zustands und der Art der Verabreichung kann die Menge der verabreichten Verbindung 0,01 µg/kg - 10 mg/kg Körpergewicht, vorzugsweise 0,04 µg/kg - 1 mg/kg Körpergewicht, je Tag betragen.
Beim Menschen entspricht dies einer Dosis von 0,8 µg bis 800 mg, vorzugsweise 3,2 µg bis 80 mg, täglich.

Eine Dosiseinheit enthält erfindungsgemäß 1,6 µg bis 200 mg einer oder mehrerer Verbindungen der allgemeinen Formel I.

Die erfindungsgemäßen Verbindungen sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemäßen Verbindungen, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche infrage, die in folgenden Literaturstellen als Hilfsstoffe für Pharmazie, Kosmetik und angrenzende Gebiete empfohlen beziehungsweise angegeben sind: Ullmans Encyklopädie der technischen Chemie, Band 4 (1953), Seite 1 bis 39; Journal of Pharmaceutical Sciences, Band 52 (1963), Seite 918 ff., H. v. Czetsch-Lindenwald, Hilfsstoffe für Pharmazie und angrenzende Gebiete; Pharm. Ind., Heft 2, 1961, Seite 72 u. ff.: Dr. H. P.

Fiedler, Lexikon der Hilfsstoffe für Pharmazie, Kosmetik und angrenzende Gebiete, Cantor KG. Aulendorf in Württemberg 1971.

Die Verbindungen können oral oder parenteral, beispielsweise intraperitoneal, intramuskulär, subkutan oder perkutan verabreicht werden. Die Verbindungen können auch in das Gewebe implantiert werden.
Zur oralen Verabreichung kommen Kapseln, Pillen, Tabletten, Dragees usw. infrage. Die Dosierungseinheiten können neben dem Wirkstoff einen pharmazeutisch verträglichen Träger, wie zum Beispiel Stärke, Zucker, Sorbit, Gelatine, Gleitmittel, Kieselsäure, Talkum usw., enthalten.
Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiermittels verwendet. Beispiele für verwendete Öle sind Olivenöl, Erdnußöl, Baumwollsamenöl, Sojabohnenöl, Rizinusöl und Sesamöl.
Die Verbindungen lassen sich auch in Form einer Depotinjektion oder eines lmplantatpräparats anwenden, die so formuliert sein können, daß eine verzögerte Wirkstoff-Freigabe ermöglicht wird.
Implantate können als inerte Materialien zum Beispiel biologisch abbaubare Polymere enthalten oder synthetische Silikone wie zum Beispiel Silikonkautschuk. Die Wirkstoffe können außerdem zur perkutanen Applikation zum Beispiel in ein Pflaster eingearbeitet werden.

Für die Herstellung von mit aktiven Verbindungen der allgemeinen Formel I beladenen Intravaginal- (z.B. Vaginalringe) oder Intrauterinsystemen (z.B. Pessare, Spiralen, IUSs, Mirena®) für die lokale Verabreichung eignen sich verschiedene Polymere wie zum Beispiel Silikonpolymere, Ethylenvinylacetat, Polyethylen oder Polypropylen.

Um eine bessere Bioverfügbarkeit des Wirkstoffes zu erreichen, können die Verbindungen auch als Cyclodextrinclathrate formuliert werden. Hierzu werden die Verbindungen mit α-, β- oder γ-Cyclodextrin oder Derivaten von diesen umgesetzt (PCT/EP95/02656 =WO-A- 96/02277).

Erfindungsgemäß können die Verbindungen der allgemeinen Formel I auch mit Liposomen verkapselt werden.

Die Verbindungen können, sowohl nach oraler als auch parenteraler Gabe, für die folgenden Indikationen eingesetzt werden.
Die erfindungsgemäßen Verbindungen besitzen ein Wirkprofil mit Hybridcharakter. Sie sind Inhibitoren der 5α-Reduktase und wirken darüber hinaus auch als Gestagene. Sie eignen sich daher zur Behandlung von Erkrankungen, die in bestimmten Organen und Geweben bei Mann und Frau Folge erhöhter Androgenspiegel sind. Bei der Frau können erhöhte Spiegel an 5α-Dihydroprogesteron zu schweren Störungen der Befindlichkeit beim prämenstruellen Syndrom beitragen. Dieses kann durch Hemmung der 5α-Reduktase günstig beeinflußt werden.
Das gleichzeitige Vorliegen einer gestagenen Wirkung bei den erfindungsgemäßen Verbindungen führt beim männlichen und weiblichen Geschlecht zu einer Hemmung der Gonadenfunktion. Dieser Effekt ist dann erwünscht, wenn mit der Behandlung eine antifertile Wirkung oder auch eine Hemmung der Hormonsekretion der Gonade erreicht werden soll. Dies ist bei Erkrankungen der Prostata (benigne Prostatahyperplasie) häufig der Fall. Mögliche Indikationen sind neben Prostataerkrankungen die Kontrazeption bei beiden Geschlechtern, Alopecie vom männlichen Typ, Akne und Hirsutismus. In Verbindung mit anderen hormonalen Substanzen, wie einem Östrogen, Testosteron bzw. einem starken Androgen, sind die erfindungsgemäßen Verbindungen geeignet als Kontrazeptiva für die Frau bzw. für den Mann. Im letzteren Fall wird die Wirkung von Testosteron in der Prostata durch Hemmung der 5α-Reduktase abgeschwächt, während die gestagene Aktivität die Wirkung in der männlichen Gonade, das heißt die Hemmung der Spermatogenese, verstärkt.

Werden die erfindungsgemäßen Verbindungen für die weibliche Kontrazeption eingesetzt, können sie allein oder in Verbindung mit einem Estrogen verwendet werden. Als Estrogene sind grundsätzlich alle estrogenwirksamen Verbindungen geeignet: Estrogene, die verwendet werden können sind beispielsweise Ethinylestradiol, 17β-Estradiol sowie dessen Ester, wie Estradiol-3-benzoat, Estradiol-17-valerat, -cypionat, - undecylat, -enanthat und/oder weitere Estradiolester (US-A-2,611,773, US-A-2,990,414, US-A-2,054,271, US-A-2,225,419 und US-A-2,156,599) und conjugierte Estrogene. Estradiol-, Ethinylestradiol- und Estron-3-sulfamate, beispielsweise Estron-N,N-dimethylsulfamat, Estron-N,N-diethylsulfamat, Ethinylestradiol-3-N,N-dimethylsulfamat, Ethinylestradiol-3-N,N-diethylsuifamat, Ethinylestradiol-3-N,N-tetramethylensulfamat, Estronsulfamat, Estradiol-3-sulfamat, Estradiol-3-N,N-dimethylsulfamat, Estradiol-3-N,N-diethylsulfamat, Ethinylestradiol-3-sulfamat, die alle Prodrugs der entsprechenden 3-Hydroxyverbindungen darstellen (W. Elger et al., in J. Steroid Biochem. Molec. Biol., Vol. 55, No. 3/4, 395-403, 1995; DE 44 29 398 A1 and DE 44 29 397 A1), können ebenso erfindungsgemäß verwendet werden.

Werden die erfindungsgemäßen Verbindungen für die männliche Kontrazeption eingesetzt, können sie allein oder in Verbindung mit Testosteron bzw. einem starken Androgen verwendet werden.

In den nachfolgenden Tabellen 1 und 2 sind Daten 5α-Reduktase-Typ2-Aktivität in Genitalhaut-Homogenaten und In vivo-Daten zur gestagenen Aktivität aufgeführt.

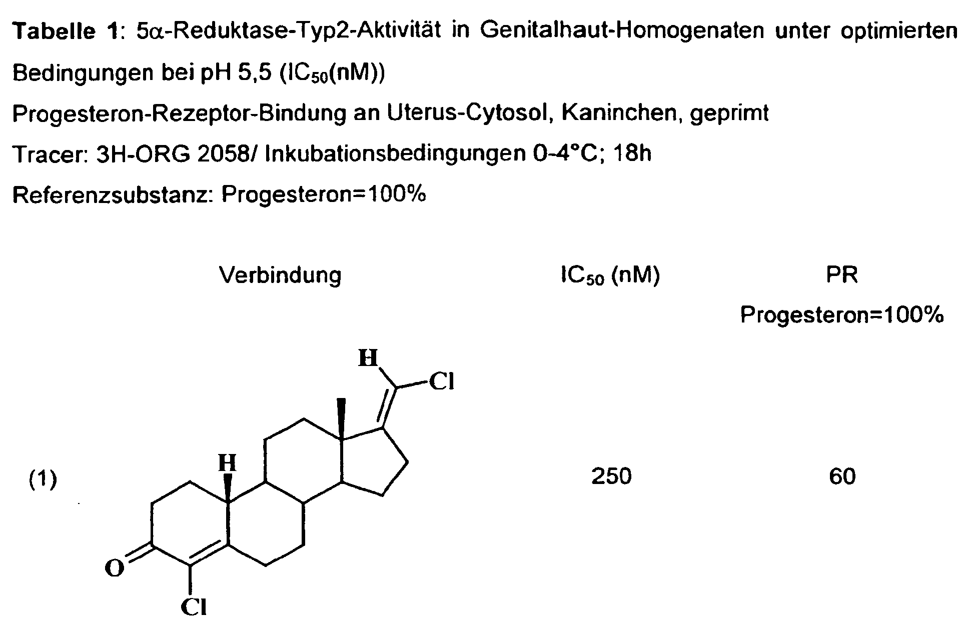

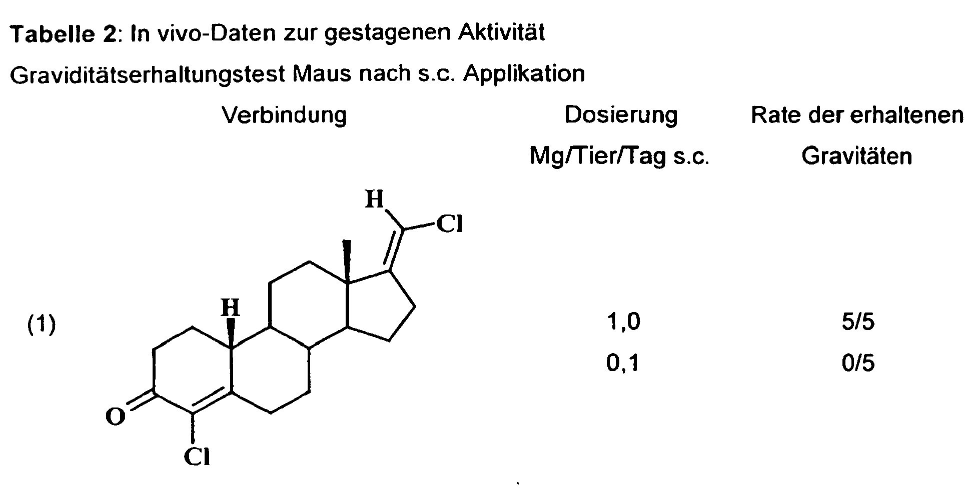

Die in Tabelle 1 und 2 aufgeführten Daten belegen das Wirkprofil mit Hybridcharakter der erfindungsgemäßen Verbindungen, die als Inhibitoren der 5α-Reduktase und als Gestagene wirken.

Die erfindungsgemäßen 17-Methylensteroide der allgemeinen Formel I sind aus Verbindungen der allgemeinen Formel II und der allgemeinen Formel V zugänglich.

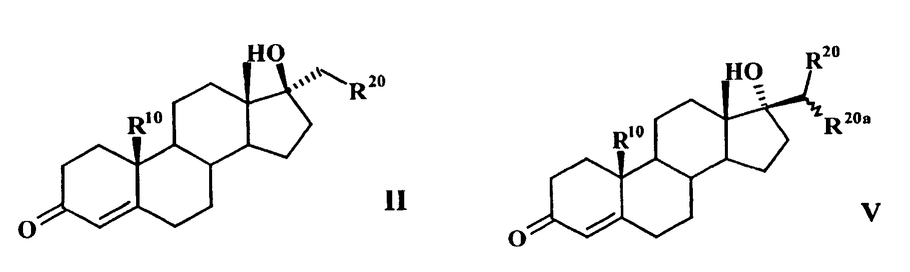

Sie werden erhalten, indem man eine Verbindung der allgemeinen Formel II in einem aprotischen Lösungsmittel, vorzugsweise in Pyridin oder Triethylamin, mit einem Säurechlorid, vorzugsweise Thionylchlorid oder Phosphoroxychlorid, zu einer Verbindung der allgemeinen Formel III umsetzt, 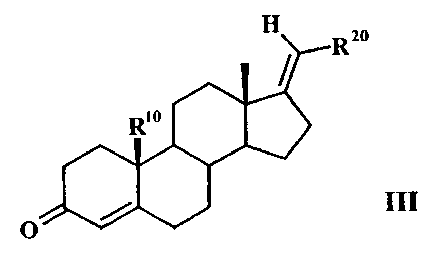 diese in an sich bekannter Weise einer Epoxidierung mit H₂O₂ / NaOH in einem Alkohol, vorzugsweise Methanol oder Ethanol, unterwirft, das resultierenden 4,5-Epoxid mit Nukleophilen, wie Halogenid (Halogenwasserstoff) oder Pseudohalogenid in einem dipolaren aprotischen Lösungsmittel, vorzugsweise DMSO oder DMPU, Dioxan oder Aceton, zu Halogen- bzw. Pseudohalogenhydrinen öffnet und diese dann unter katalytischer Vermittlung durch Mineralsäure, Carbonsäure oder Sulfonsäure, wie Chlorwasserstoffsäure, Oxalsäure oder p-Toluolsulfonsäure, in einem protischen oder aprotischen Lösungsmittel, wie Methanol oder Aceton, zu einer Verbindung der allgemeinen Formel IV dehydratisiert 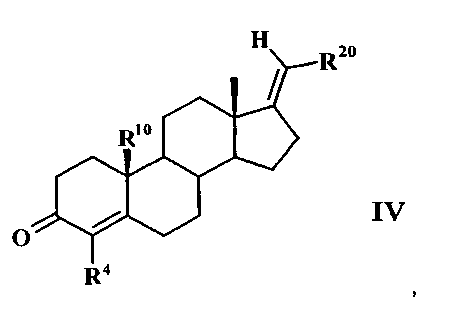
worin R⁴ für ein Halogenatom oder Pseudohalogen steht, wobei R¹⁰ die vorstehend gegebene Bedeutung aufweist und R²⁰ eine C₁-₄-Alkyl- oder Hydroxy-C₁₋₄-alkylgruppe, ein Halogenatom oder ein Pseudohalogen bedeutet.

Es werden weiterhin 17-Methylensteroide der allgemeinen Formel I erhalten, indem man eine Verbindung der allgemeinen Formel V in einem aprotischen Lösungsmittel, vorzugsweise in Pyridin oder Triethylamin, mit einem Säurechlorid, vorzugsweise Thionylchlorid oder Phosphoroxychlorid, zu einer Verbindung der allgemeinen Formel VI umsetzt, 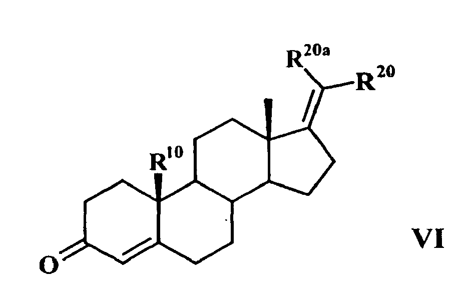 diese in an sich bekannter Weise einer Epoxidierung mit H₂O₂ / NaOH in einem Alkohol, vorzugsweise Methanol oder Ethanol, unterwirft, das resultierende 4,5-Epoxid mit einem Nukleophil, wie einem Halogenid (Halogenwasserstoff) oder Pseudohalogenid in einem dipolaren aprotischen Lösungsmittel, vorzugsweise DMSO oder DMPU, Dioxan oder Aceton, zu einem Halogen- bzw. Pseudohalogenhydrin öffnet und dieses dann unter katalytischer Vermittlung durch Mineralsäure, Carbonsäure oder Sulfonsäure, wie Chlorwasserstoffsäure, Oxalsäure oder p-Toluolsulfonsäure, in einem protischen oder aprotischen Lösungsmittel, Methanol oder Aceton, zu einer Verbindung der allgemeinen Formel VII dehydratisiert.

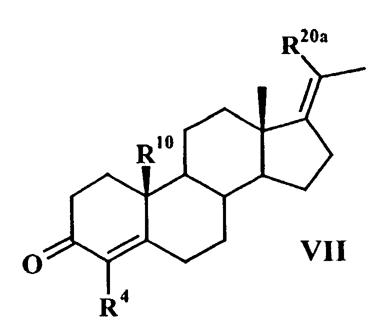

Schließlich können weitere 17-Methylensteroide der allgemeinen Formel I erhalten werden, indem man Verbindungen der allgemeinen Formeln III und VI 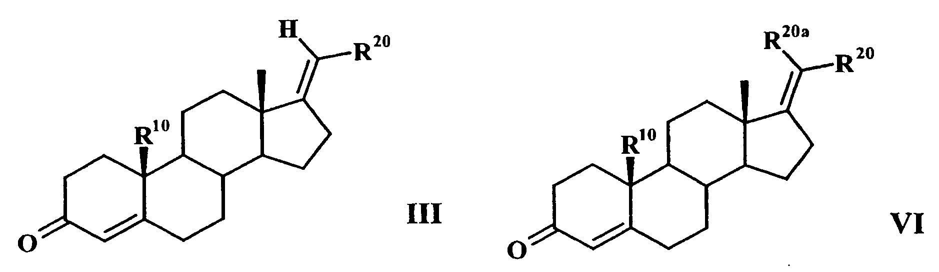 in an sich bekannter Weise (M. Haase-Held, M. Mattis u. J. Mann, J. Chem. Soc. Perkin Trans. 1, 2999, 1992) mit NalO₄/KMnO₄ in Alkoholen, vorzugsweise in t.-BuOH zu den 3,5-Seco-Ketosäuren umsetzt, diese mit AC₂O/AcCl in die ungesättigten Lactone überführt und diese mit n-BuLi/CH₃CN in einem dipolaren aprotischen Lösungsmittel wie THF zu den Verbindungen der allgemeinen Formeln VIII und IX umsetzt.

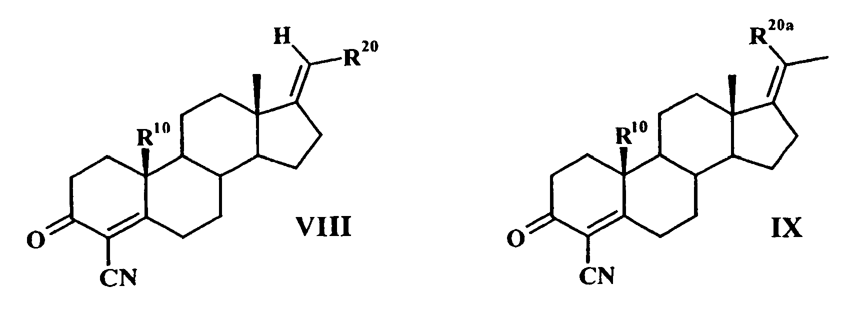

Verbindungen der Formeln III und IV können auch durch Carbonylolefinierung nach WITTIG, HORNER, PETERSON erhalten werden.

Die Erfindung wird durch die nachfolgenden Beispielen näher erläutert.

### Beispiel 1

### Z-4,20-Dichlor-19-norpregna-4,17(20)-dien-3-on

### A) Z-20-Chlor-19-norpregna-4,17(20)-dien-3-on

9,74 mmol (3,28 g) (20)R-Chlor-19-norpregn-4-en-3-on-17-ol werden in 34 ml Pyridin gelöst. Unter leichter Kühlung werden unter Rühren 12,66 mmol (0,92 ml) Thionylchlorid zugetropft. Unter Schutzgas (Argon) wird ca. 1 Stunde gerührt und die Reaktionslösung anschließend in eisgekühltes, mit Salzsäure auf pH=3-4 eingestelltes Wasser gegeben. Das klebrige Fällungsprodukt wird mit Methylenchlorid extrahiert, die vereinigten, neutralgewaschenen Extrakte mit Natriumsulfat getrocknet und eingeengt. Das anfallende Festprodukt wird durch Flash-Chromatographie an Kieselgel (Eluent: Toluen/Essigester = 95/5) und gegebenenfalls Umkristallisation aus Methanol gereinigt.
Man erhält 0,6 g eines festen Produktes.
Fₚ=138-141°C; [α]_{D}²⁰=+104° (CHCl₃)

### B) Z-20-Chlor-4ξ,5ξ-epoxy-19-norpregn-17(20)-en-3-on

2,80 mmol (894 mg) Z-20-Chlor-19-norpregna-4,17(20)-dien-3-on werden in einer Mischung von 9 ml Methanol und 7,3 ml Methylenchlorid gelöst und auf 0°C abgekühlt. Zur gekühlten Lösung werden nacheinander unter Rühren und Schutzgas (Argon) 6,31 mmol (0,63 ml) Wasserstoffperoxid (30%-ig) und 1,26 mmol (0,3 ml) Natronlauge (c=4 mol/l) getropft. Nach beendetem Zutropfen wird die Temperatur langsam auf Raumtemperatur gesteigert. Nach ca. 1,5 Stunden Reaktionszeit wird die Mischung auf Eiswasser gegeben und danach mit Methylenchlorid extrahiert. Die vereinigten Extrakte werden mit Natriumsulfat getrocknet und eingeengt. Man erhält 926 mg eines hellgelben Feststoffes.

### C) Z-4,20-Dichlor-19-norpregna-4,17(20)-dien-3-on

2,71 mmol (896 mg) Z-20-Chlor-4ξ,5ξ-epoxy-19-norpregn-17(20)-en-3-on werden in 25 ml 1,3-Dimethyltetrahydro-2(1H)-pyrimidinon (DMPU) gelöst und langsam 26,54 mmol (2,2 ml) Salzsäure (37%-ig) zugetropft. Nach 1 Stunde Rühren unter Schutzgas (Argon) wird die Reaktionslösung auf eisgekühlte, wäßrige Natriumhydrogencarbonatlösung gegeben, die Fällung abgesaugt und schließlich im Exsikkator mit Kaliumhydroxid getrocknet. Durch Flash-Chromatographie an Kieselgel (Eluent: Toluen/Essigester = 99/1) und anschließende Umkristallisation aus Methanol/Aceton erhält man 353 mg festes Produkt.
Fp=182-185°C; [α]_{D}²⁰=+137° (CHCl₃)

### Beispiel 2

### Z-20-Brom-4-chlor-19-norpregna-4,17(20)-dien-3-on

### A) Z-20-Brom-19-norpregna-4,17(20)-dien-3-on

9,7 mmol (3,7 g) (20)R-Brom-19-norpregn-4-en-3-on-17-ol werden in 39 ml Pyridin gelöst. Unter leichter Kühlung werden unter Rühren 12,61 mmol (0,91 ml) Thionylchlorid zugetropft. Unter Schutzgas (Argon) wird ca. 1 Stunde gerührt und die Reaktionslösung anschließend in eisgekühltes, mit Salzsäure auf pH=3-4 eingestelltes Wasser gegeben. Das klebrige Fällungsprodukt wird mit Methylenchlorid extrahiert, die vereinigten, neutralgewaschenen Extrakte mit Natriumsulfat getrocknet und eingeengt. Der anfallende gelbe Schaum wird durch Flash-Chromatographie an Kieselgel (Eluent: n-Hexan/Essigester = 95/5) und gegebenenfalls Umkristallisation aus Methanol/Aceton gereinigt. Man erhält 536 mg eines hellgelben Feststoffes.
Fp=153-158°C; [α]_{D}²⁰=+109° (CHCl₃)

### B) Z-20-Brom-4ξ,5ξ-epoxy-19-norpregn-17(20)-en-3-on

2,09 mmol (760 mg) Z-20-Brom-19-norpregna-4,17(20)-dien-3-on werden in einer Mischung von 7,8 ml Methanol und 6,3 ml Methylenchlorid gelöst und auf 0°C abgekühlt. Zur gekühlten Lösung werden nacheinander unter Rühren und Schutzgas (Argon) 4,7 mmol (0,48 ml) Wasserstoffperoxid (30%-ig) und 0,94 mmol (0,24 ml) Natronlauge (c=4 mol/l) getropft. Nach beendetem Zutropfen wird die Temperatur langsam auf Raumtemperatur gesteigert. Nach ca. 1 Stunde Reaktionszeit werden zu der Mischung 50 ml Wasser gegeben. Es entsteht ein Zwei-Phasen-System, welches mit Methylenchlorid extrahiert wird. Die vereinigten, neutralgewaschenen Extrakte werden mit Natriumsulfat getrocknet und eingeengt. Das anfallende gelbe Festprodukt wird durch Flash-Chromatographie an Kieselgel (Eluent: n-Hexan/Essigester = 95/5) gereinigt. Man erhält 680 mg eines weißen Festproduktes.

### C) Z-20-Brom-4-chlor-19-norpregna-4,17(20)-dien-3-on

2,57 mmol (975 mg) Z-20-Brom-4ξ,5ξ-epoxy-19-norpregn-17(20)en-3-on werden in 25 ml DMPU gelöst und langsam 25,2 mmol (2,09 ml) Salzsäure (37%-ig) zugetropft. Nach 1 Stunde Rühren unter Schutzgas (Argon) wird die Reaktionslösung auf eisgekühlte, wäßrige Natriumhydrogencarbonatlösung gegeben, die Fällung abgesaugt und schließlich im Exsikkator mit Kaliumhydroxid getrocknet. Durch Flash-Chromatographie an Kieselgel (Eluent Toluen) und anschließende Umkristallisation aus Methanol/Aceton erhält man 600 mg weiße Kristalle.
Fp=164-173°C; [α]_{D}²⁰=+138° (CHCl₃)

### Beispiel 3

### E-17-Chlormethylen-4-chlor-estr-4-en-3-on

### A) E-17-Chlormethylen-estr-4-en-3-on

18,27 mmol (5,9 g) 17α-Chlormethyl-17-hydroxy-estr-4-en-3-on werden in 60 ml Pyridin gelöst. Unter leichter Kühlung werden unter Rühren 21,9 mmol (1,56 ml) Thionylchlorid zugetropft. Unter Schutzgas (Argon) wird ca. 1 Stunde gerührt und die Reaktionslösung anschließend in eisgekühltes, mit Salzsäure auf pH=3-4 eingestelltes Wasser gegeben. Das klebrige Fällungsprodukt wird mit Methylenchlorid extrahiert, die vereinigten, neutralgewaschenen Extrakte über Natriumsulfat getrocknet und eingeengt. Das anfallende Festprodukt wird durch Flash-Chromatographie an Kieselgel (Eluent: n-Hexan/Essigester = 85/15) gereinigt. Nach Umkristallisation aus Aceton erhält man 1,6 g Produkt.
Fp=143-146°C; [α]_{D}²⁰=+20° (CHCl₃)

### B) E-17-Chlormethylen-4ξ,5ξ-epoxy-estran-3-on

3,93 mmol (1,2 g) E-17-Chlormethylen-estr-4-en-3-on werden in einer Mischung von 12 ml Methanol und 10 ml Methylenchlorid unter Schutzgas (Argon) gelöst und auf 0°C abgekühlt. Zur gekühlten Lösung werden nacheinander unter Rühren 9,2 mmol (0,94 ml) Wasserstoffperoxid (30%-ig) und 1,65 mmol (0,4 ml) Natronlauge (c=4 mol/l) zugetropft.

Nach beendetem Zutropfen wird die Temperatur langsam auf Raumtemperatur gesteigert. Nach ca. 1,5 Stunden Reaktionszeit wird die Mischung auf Eiswasser gegeben und mit Methylenchlorid extrahiert. Die vereinigten Extrakte werden über Natriumsulfat getrocknet und eingeengt. Man erhält 1,1 g eines weißen, klebrigen Schaums.

### C) E 17-Chlormethylen-4-chlor-estr-4-en-3-on

3,42 mmol (1,1 g) E-17-Chlormethylen-4ξ,5ξ-epoxy-estran-3-on werden in 26 ml DMPU gelöst und langsam 33 mmol (2,74 ml) Salzsäure (37%-ig) zugetropft. Nach 1 Stunde wird die Reaktionslösung auf eisgekühlte wässrige Natriumhydrogencarbonatlösung gegeben, die Fällung abgesaugt und im Exsikkator über Kaliumhydroxid getrocknet. Nach Umkristallisation aus Aceton erhält man 760 mg weiße Kristalle.
Fp=182-194°C; [α]_{D}²⁰=+63° (CHCl₃)

### Beispiel 4

### E-17-Brommethylen-4-chlor-estr-4-en-3-on

### A) E-17-Brommethylen-estr-4-en-3-on

28,58 mmol (10,5 g) 17α-Brommethyl-17-hydroxy-estr-4-en-3-on werden in 90 ml Pyridin gelöst. Unter leichter Kühlung werden unter Rühren 37,16 mmol (2,7 ml) Thionylchlorid zugetropft. Unter Schutzgas (Argon) wird ca. 1 Stunde gerührt und die Reaktionslösung anschließend in eisgekühltes, mit Salzsäure auf pH=3-4 eingestelltes Wasser gegeben. Das gelbe Fällungsprodukt wird abgesaugt und durch Flash-Chromatographie an Kieselgel (Eluent: n-Hexan/Essigester = 85/15) gereinigt. Man erhält 4,85 g schwach gelbgefärbten Feststoff. Nach Umkristallisation aus Aceton erhält man 3,1 g weiße Kristalle.
Fp=153-164°C; [α]_{D}²⁰=+15° (CHCl₃)

### B) E-17-Brommethylen-4ξ,5ξ-epoxy-estran-3-on

2,86 mmol (1 g) E-17-Brommethylen-estr-4-en-3-on werden in einer Mischung von 10,5 ml Methanol und 8,5 ml Methylenchlorid unter Schutzgas (Argon) gelöst und auf 0°C abgekühlt. Zur gekühlten Lösung werden nacheinander unter Rühren 6,44 mmol (0,66 ml) Wasserstoffperoxid (30%-ig) und 1,26 mmol (0,32 ml) Natronlauge (c=4 mol/l) zugetropft. Nach beendetem Zutropfen wird die Temperatur langsam auf Raumtemperatur gesteigert. Nach ca. 1,5 Stunden Reaktionszeit wird die Mischung auf Eiswasser gegeben. Es entsteht ein Zwei-Phasen-System, welches mit Methylenchlorid extrahiert wird. Die vereinigten, neutralgewaschenen Extrakte werden mit Natriumsulfat getrocknet und eingeengt. Man erhält 891 mg eines weißen, klebrigen Schaums.

### C. E-17-Brommethylen-4-chlor-estr-4-en-3-on

2,38 mmol (ca. 870 mg) E-17-Brommethylen-4ξ,5ξ-epoxy-estran-3-on werden in 23 ml DMPU unter Schutzgas (Argon) gelöst und langsam 23,33 mmol (1,93 ml) Salzsäure (37%-ig) zugetropft. Nach 1 Stunde wird die Reaktionslösung auf eisgekühlte, wäßrige Natriumhydrogencarbonatlösung gegeben, die Fällung abgesaugt und im Exsikkator mit Kaliumhydroxid getrocknet. Durch Flash-Chromatographie an Kieselgel (Eluent: Toluen/Essigester = 98/2) und anschließende Umkristallisation aus Aceton erhält man 552 mg weiße Kristalle.
Fp=169-176°C; [α]_{D}²⁰=+45° (CHCl₃)

## Patentansprüche

1. 17-Methylensteroide der allgemeinen Formel I 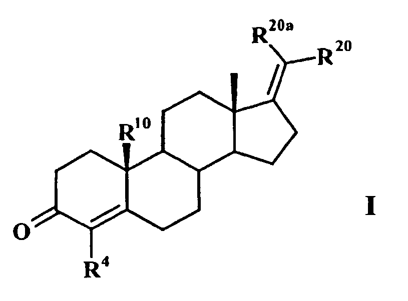
worin
R⁴ für ein Halogenatom oder ein Pseudohalogen steht,
R¹⁰ für ein Wasserstoffatom oder eine geradkettige oder verzweigte C-₁₋₄₋Alkylgruppe steht,
R²⁰ und R^{20a} unabhängig voneinander ein Wasserstoffatom, eine geradkettige oder verzweigte C₁₋₄-Alkyl- oder Hydroxy-C₁₋₄-alkylgruppe darstellen oder
einer der Reste R²⁰ und R^{20a} ein Wasserstoffatom, eine geradkettige oder verzweigte C₁₋₄-Alkyl- oder Hydroxy-C₁₋₄-alkylgruppe bedeutet und der andere Rest ein Halogenatom oder ein Pseudohalogen bedeutet mit Ausnahme der Verbindungen: 4-chloro-17-methylen-4-androsten-3-on und 4-chloro-20ξ-fluoro-21-hydroxypregna-4,17(20)-dien-3-on.

2. 17-Methylensteroide nach Anspruch 1, **dadurch gekennzeichnet, daß** R⁴ für ein Chlor- oder Bromatom oder eine Cyanogruppe steht.

3. 17-Methylensteroide nach Anspruch 1 oder 2, **dadurch gekennzeichnet, daß** einer der Reste R²⁰ und R^{20a} ein Wasserstoffatom oder eine Methylgruppe bedeutet und der andere Rest ein Fluor-, Chlor- oder Bromatom, eine Azido-, Cyano- oder Rhodanogruppe oder Hydroxymethyl bedeutet.

4. 17-Methylensteroide nach Anspruch 1, 2 oder 3, **dadurch gekennzeichnet, daß** R¹⁰ für ein Wasserstoffatom oder eine Methylgruppe steht.

5. 17-Methylensteroide nach Anspruch 1, nämlich
15) E-17-Chlormethylen-4-chlor-estr-4-en-3-on,
16) E-17-Cyanomethylen-4-chlor-estr-4-en-3-on,
17) Z-17-Cyanomethylen-4-chlor-estr-4-en-3-on,
18) Z-17-(1')-Cyanoethyliden-4-chlor-estr-4-en-3-on,
19) Z-17-Ethyliden-4-chlor-estr-4-en-3-on,
20) E-17-Ethyliden-4-chlor-estr-4-en-3-on,
21) E-17-Brommethylen-4-chlor-estr-4-en-3-on,
22) Z-17-Chiorethyliden-4-chlor-estr-4-en-3-on,
23) Z-17-Bromethyliden-4-chlor-estr-4-en-3-on,
24) E-17-Chlormethylen-4-cyano-androst-4-en-3-on,
25) E-17-Chlormethylen-4-chlor-androst-4-en-3-on,
26) E-17-(2')-Hydroxyethyliden -4-chlor-estr-4-en-3-on und
27) Z-17-(2')-Hydroxyethyliden -4-chlor-estr-4-en-3-on.

6. Verfahren zur Herstellung von 17-Methylensteroiden nach Anspruch 1, worin R^{20a} ein Wasserstoffatom bedeutet, **dadurch gekennzeichnet, daß** man eine Verbindung der allgemeinen Formel II, 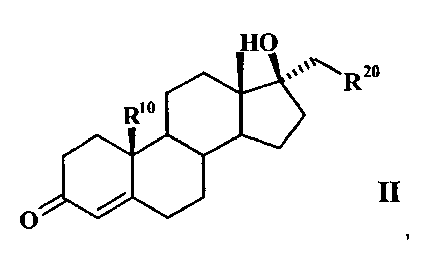
in einem aprotischen Lösungsmittel mit einem Säurechlorid zu einem 17-Methylensteroid der allgemeinen Formel III umsetzt, 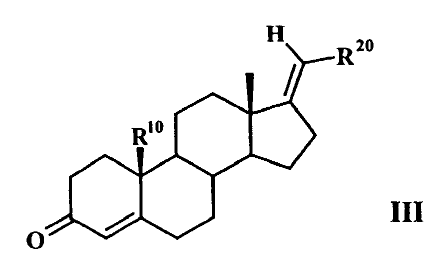
mit H₂O₂/NaOH das 4,5-Epoxid generiert, dieses dann mit einem nukleophilen Reagenz, das sich von einem Halogenatom bzw. Pseudohalogen ableitet, in einem dipolaren aprotischen Lösungsmittel zu einem Halogen- bzw. Pseudohalogenhydrin öffnet und gegebenenfalls mit Mineralsäure, Carbonsäure oder Sulfonsäure in einem protischen oder aprotischen Lösungsmittel unter Dehydratisierung zu einer Verbindung der allgemeinen Formel IV umsetzt, worin 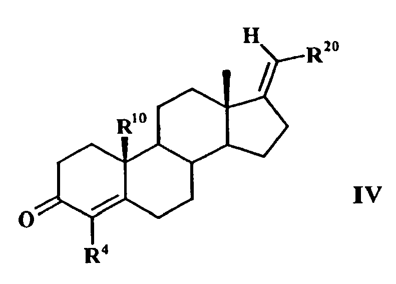
R⁴ für ein Halogenatom oder Pseudohalogen steht, wobei R¹⁰ die in Anspruch 1 gegebene Bedeutung aufweist und R²⁰ eine C₁₋₄-Alkyl- oder Hydroxy-C₁₋₄₋alkylgruppe, ein Halogenatom oder ein Pseudohalogen bedeutet.

7. Verfahren zur Herstellung von 17-Methylensteroiden nach Anspruch 1, **dadurch gekennzeichnet, daß** man eine Verbindung der allgemeinen Formel V, 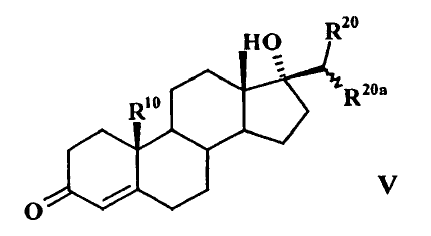
mit einem Säurechlorid in einem aprotischen Lösungsmittel zu einem Methylensteroid der allgemeinen Formel VI umsetzt, 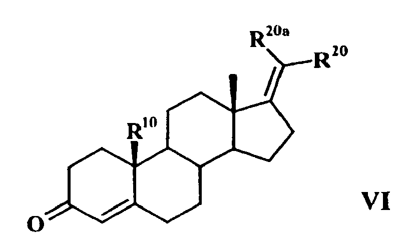
mit H₂O₂ / NaOH das 4,5-Epoxid generiert, dieses dann mit einem nukleophilen Reagenz, das sich von einem Halogenatom bzw. Pseudohalogen ableitet, in einem dipolaren aprotischen Lösungsmittel zu einem Halogen- bzw. Pseudohalogenhydrin öffnet und gegebenenfalls mit Mineralsäure, Carbonsäure oder Sulfonsäure in einem protischen oder aprotischen Lösungsmittel unter Dehydratisierung zu einer Verbindung der allgemeinen Formel VII umsetzt, 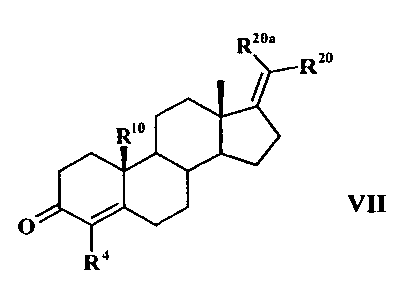
worin R⁴ für ein Halogenatom oder Pseudohalogen steht, wobei R¹⁰ die in Anspruch 1 gegebene Bedeutung aufweist und R²⁰ eine C₁₋₄-Alkyl- oder Hydroxy-C₁₋₄-alkylgruppe bedeutet und R^{20a} ein Wasserstoffatom, ein Halogenatom oder ein Pseudohalogen, bedeutet.

8. Pharmazeutische Zusammensetzungen die mindestens ein 17-Methylensteroid nach Anspruch 1, 2, 3, 4 oder 5 enthalten, gegebenenfalls zusammen mit pharmazeutisch verträglichen Hilfs- und Trägerstoffen.

9. 17-Methylensteroide nach einem der Ansprüche 1 bis 5 zur Anwendung als therapeutische Wirkstoffe.

10. Verwendung der Verbindungen nach einem der Ansprüche 1 bis 5 zur Herstellung eines Medikaments zur Behandlung von Prostataerkrankungen, Alopecie vom männlichen Typ, Akne und Hirsutismus, zur Kontrazeption bei Mann und Frau und/oder zur Inhibition der 5α-Reduktase.

## Claims

1. 17-Methylenesteroids of the general formula I 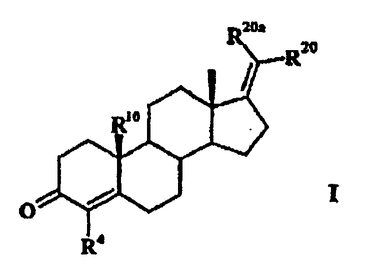
in which
R⁴ is a halogen atom or a pseudohalogen,
R¹⁰ is a hydrogen atom or a straight-chain or branched C₁₋₄-alkyl group,
R²⁰ and R^{20a} are, independently of one another, a hydrogen atom, a straight-chain or branched C₁₋₄₋alkyl or hydroxy-C₁₋₄-alkyl group or
one of the radicals R²⁰ and R^{20a} is a hydrogen atom, a straight-chain or branched C₁₋₄-alkyl or hydroxy-C₁₋₄-alkyl group, and the other radical is a halogen atom or a pseudohalogen, with the exception of the following compounds: 4-chloro-17-methylene-4-androsten-3-one and 4-chloro-20ξ-fluoro-21-hydroxypregna-4,17(20)-dien-3-one.

2. 17-Methylenesteroids according to Claim 1, **characterized in that** R⁴ is a chlorine or bromine atom or a cyano group.

3. 17-Methylenesteroids according to Claim 1 or 2, **characterized in that** one of the radicals R²⁰ and R^{20a} is a hydrogen atom or a methyl group, and the other radical is a fluorine, chlorine or bromine atom, an azido, cyano or thiocyanato group or hydroxymethyl.

4. 17-Methylenesteroids according to Claim 1, 2 or 3, **characterized in that** R¹⁰ is a hydrogen atom or a methyl group.

5. 17-Methylenesteroids according to Claim 1, namely
1) E-17-chloromethylene-4-chloroestr-4-en-3-one,
2) E-17-cyanomethylene-4-chloroestr-4-en-3-one,
3) Z-17-cyanomethylene-4-chloroestr-4-en-3-one,
4) Z-17-(1')-cyanoethylidene-4-chloroestr-4-en-3-one,
5) Z-17-ethylidene-4-chloroestr-4-en-3-one,
6) E-17-ethylidene-4-chloroestr-4-en-3-one,
7) E-17-bromomethylene-4-chloroestr-4-en-3-one,
8) Z-17-chloroethylidene-4-chloroestr-4-en-3-one,
9) Z-17-bromoethylidene-4-chloroestr-4-en-3-one,
10) E-17-chloromethylene-4-cyanoandrost-4-en-3-one,
11) E-17-chloromethylene-4-chloroandrost-4-en-3-one,
12) E-17-(2')-hydroxyethylidene-4-chloroestr-4-en-3-one and
13) Z-17-(2')-hydroxyethylidene-4-chloroestr-4-en-3-one.

6. Process for preparing 17-methylenesteroids according to Claim 1, in which R^{20a} is a hydrogen atom, **characterized in that** a compound of the general formula II 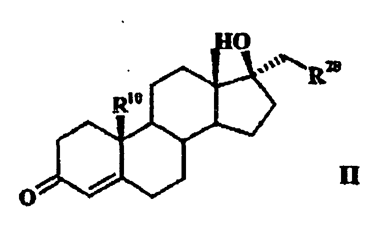
is reacted in an aprotic solvent with an acid chloride to give a 17-methylenesteroid of the general formula III, 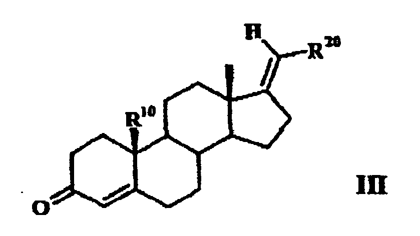
the 4,5-epoxide is generated with H₂O₂/NaOH, the latter is then opened with a nucleophilic reagent which is derived from a halogen atom or pseudohalogen, in a dipolar aprotic solvent, to give a halo- or pseudohalohydrin and, where appropriate, reacted with mineral acid, carboxylic acid or sulphonic acid in a protic or aprotic solvent with dehydration to give a compound of the general formula IV, in which 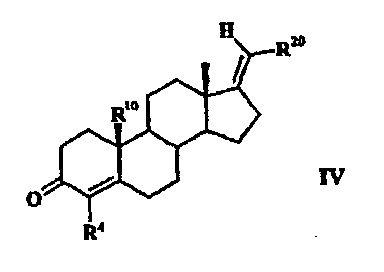
R⁴ is a halogen atom or pseudohalogen, where R¹⁰ has the meaning given in Claim 1, and R²⁰ is a C₁₋₄₋alkyl or hydroxy-C₁₋₄-alkyl group, a halogen atom or a pseudohalogen.

7. Process for preparing 17-methylenesteroids according to Claim 1, **characterized in that** a compound of the general formula V 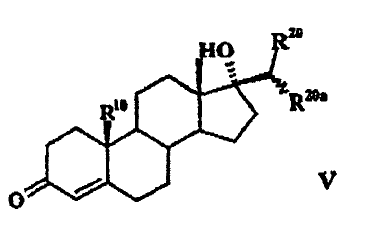
is reacted with an acid chloride in an aprotic solvent to give a methylenesteroid of the general formula VI, 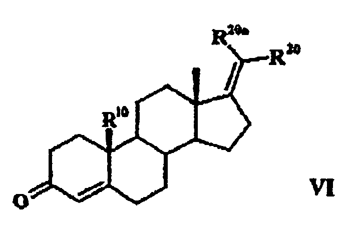
the 4,5-epoxide is generated with H₂O₂/NaOH, the latter is then opened with a nucleophilic reagent which is derived from a halogen atom or pseudohalogen, in a dipolar aprotic solvent, to give a halo- or pseudohalohydrin and, where appropriate, reacted with mineral acid, carboxylic acid or sulphonic acid in a protic or aprotic solvent with dehydration to give a compound of the general formula VII 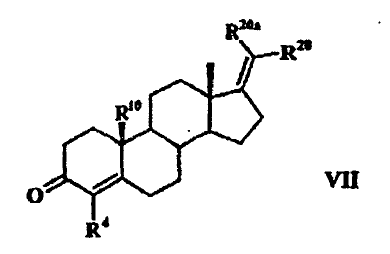
in which R⁴ is a halogen atom or pseudohalogen, where R¹⁰ has the meaning given in Claim 1, and R²⁰ is a C₁₋₄-alkyl or hydroxy-C₁₋₄-alkyl group, and R^{20a} is a hydrogen atom, a halogen atom or a pseudohalogen.

8. Pharmaceutical compositions comprising at least one 17-methylenesteroid according to Claim 1, 2, 3, 4 or 5, where appropriate together with pharmaceutically acceptable excipients and carriers.

9. 17-Methylenesteroids according to any of Claims 1 to 5 for use as therapeutic active ingredients.

10. Use of the compounds according to any of Claims 1 to 5 for the production of a medicament for the treatment of prostate disorders, male-type alopecia, acne and hirsutism, for contraception in men and women and/or for inhibition of 5α-reductase.

## Revendications

1. Stéroïdes 17-méthyléniques de la formule générale 1 : 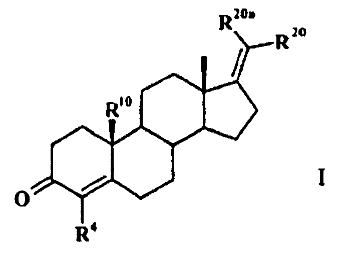
dans laquelle
R⁴ représente un atome d'halogène ou un pseudohalogène,
R¹⁰ représente un atome d'hydrogène ou un groupe alkyle en C₁-C₄ linéaire ou ramifié,
R²⁰ et R^{20a} représentent, indépendamment l'un de l'autre, un atome d'hydrogène, un groupe alkyle en C₁-C₄ ou hydroxyalkyle en C₁-C₄, linéaire ou ramifié, ou un des radicaux R²⁰ et R^{20a} représente un atome d'hydrogène, un groupe alkyle en C₁-C₄ ou hydroxyalkyle en C₁-C₄, linéaire ou ramifié, et l'autre radical un atome d'halogène ou un pseudohalogène,
à l'exception des composés : 4-chloro-17-méthylène-4-androstén-3-one et 4-chloro-20ξ-fluoro-21-hydroxyprégna-4,17(20)-dièn-3-one.

2. Stéroïdes 17-méthyléniques suivant la revendication 1, **caractérisés en ce que** R⁴ représente un atome de chlore ou de brome ou un groupe cyano.

3. Stéroïdes 17-méthyléniques suivant la revendication 1 ou 2, **caractérisés en ce qu'**un des radicaux R²⁰ et R^{20a} représente un atome d'hydrogène ou un groupe méthyle et l'autre radical un atome de fluor, de chlore ou de brome, un groupe azido, cyano ou rhodano ou un groupe hydroxyméthyle.

4. Stéroïdes 17-méthyléniques suivant la revendication 1, 2 ou 3, **caractérisés en ce que** R¹⁰ représente un atome d'hydrogène ou un groupe méthyle.

5. Stéroïdes 17-méthyléniques suivant la revendication 1, à savoir
15) E-17-chlorométhylène-4-chloro-oestr-4-én-3-one,
16) E-17-cyanométhylène-4-chloro-oestr-4-én-3-one,
17) Z-17-cyanométhylène-4-chloro-oestr-4-én-3-one,
18) Z-17-(1')-cyanoéthylidène-4-chloro-oestr-4-én-3-one,
19) Z-17-éthylidène-4-chloro-oestr-4-én-3-one,
20) E-17-éthylidène-4-chloro-oestr-4-én-3-one,
21) E-17-bromométhylène-4-chloro-oestr-4-én-3-one,
22) Z-17-chloroéthylidène-4-chloro-oestr-4-én-3-one,
23) Z-17-btomoéthylidène-4-chloro-oestr-4-én-3-one,
24) E-17-chlorométhylène-4-cyano-androst-4-én-3-one,
25) E-17-chlorométhylène-4-chloro-androst-4-én-3-one,
26) E-17-(2')-hydroxyéthylidène-4-chloro-oestr-4-én-3-one, et
27) Z-17-(2')-hydroxyéthylidène-4-chloro-oestr-4-én-3-one.

6. Procédé de préparation de stéroïdes 17-méthyléniques suivant la revendication 1, dans lequel R^{20a} représente un atome d'hydrogène, **caractérisé en ce qu'**on fait réagir un composé de la formule générale II : 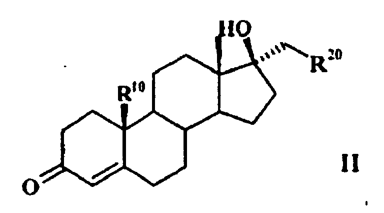
dans un solvant aprotique avec un chlorure d'acide pour former un stéroïde 17-méthylénique de la formule générale III : 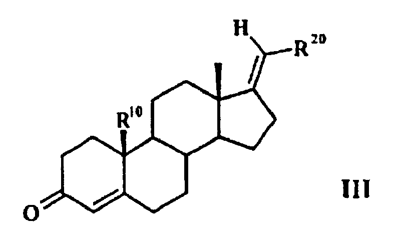
on forme le 4,5-époxyde avec H₂O₂/NaOH, on ouvre ensuite celui-ci avec un réactif nucléophile, qui dérive d'un atome d'halogène ou d'un pseudohalogène, dans un solvant aprotique dipolaire pour former une halogénhydrine ou pseudohalogénhydrine et éventuellement on fait réagir avec un acide minéral, un acide carboxylique ou un acide sulfonique dans un solvant protique ou aprotique, tout en déshydratant, ce qui donne un composé de la formule générale IV : 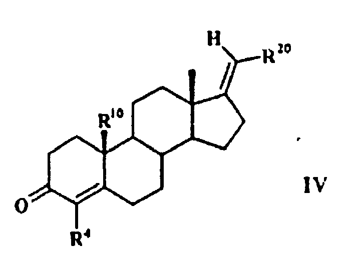
où R⁴ représente un atome d'halogène ou un pseudohalogène, R¹⁰ présente la signification indiquée dans la revendication 1 et R²⁰ représente un groupe alkyle en C₁-C₄ ou hydroxyalkyle en C₁-C₄, un atome d'halogène ou un pseudohalogène.

7. Procédé de préparation de stéroïdes 17-méthyléniques suivant la revendication 1, **caractérisé en ce qu'**on fait réagir un composé de la formule générale V : 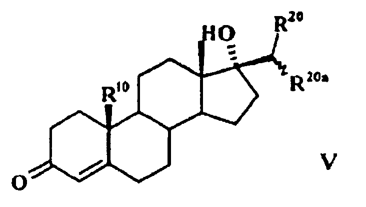
avec un chlorure d'acide dans un solvant aprotique pour former un stéroïde méthylénique de la formule générale VI : 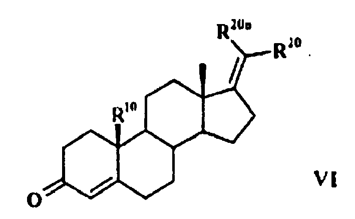
on produit le 4,5-époxyde avec H₂O/NaOH on ouvre ensuite celui-ci avec un réactif nucléophile, qui dérive d'un atome d'halogène ou d'un pseudohalogène, dans un solvant aprotique dipolaire pour former une halogénhydrine ou pseudohalogénhydrine et éventuellement on fait réagir avec un acide minéral, un acide carboxylique ou un acide sulfonique dans un solvant protique ou aprotique, tout en déshydratant, ce qui donne un composé de la formule générale VII : 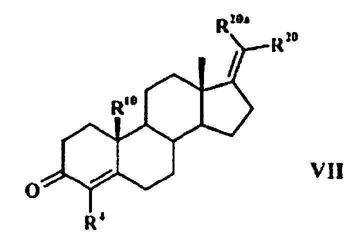
dans laquelle R⁴ représente un atome d'halogène ou un pseudohalogène, R¹⁰ présente la signification donnée dans la revendication 1 et R²⁰ représente un groupe alkyle en C₁-C₄ ou hydroxyalkyle en C₁-C₄ et R^{20a} représente un atome d'hydrogène, un atome d'halogène ou un pseudohalogène.

8. Compositions pharmaceutiques qui contiennent un stéroïde 17-méthylénique suivant la revendication 1, 2, 3, 4 ou 5, éventuellement conjointement à des adjuvants et supports pharmaceutiquement compatibles.

9. Stéroïdes 17-méthyléniques suivant l'une des revendications 1 à 5, pour l'application comme substances actives thérapeutiques.

10. Utilisation des composés suivant l'une des revendications 1 à 5, pour la fabrication d'un médicament destiné au traitement de maladies de la prostate, d'alopécie du type humain, d'acné et d'hirsutisme, pour la contraception chez l'homme et la femme et/ou pour l'inhibition de la 5α-réductase.
